## Europäisches Patentamt
## European Patent Office
## Office européen des brevets

(11) Publication number: **0 211 946**
**B1**

# EUROPEAN PATENT SPECIFICATION

(45) Date of publication of patent specification: **23.05.90**

(51) Int. Cl.⁵: **A 61 K 33/14, A 61 K 9/52**

(21) Application number: **86901631.1**

(22) Date of filing: **19.02.86**

(86) International application number:
**PCT/US86/00318**

(87) International publication number:
**WO 86/04817 28.08.86 Gazette 86/19**

(54) **CONTROLLED RELEASE POTASSIUM CHLORIDE.**

(30) Priority: **19.02.85 US 702714**

(43) Date of publication of application:
**04.03.87 Bulletin 87/10**

(45) Publication of the grant of the patent:
**23.05.90 Bulletin 90/21**

(84) Designated Contracting States:
**AT BE CH DE FR GB IT LI LU NL SE**

(56) References cited:
**EP-A-0 052 076**
**WO-A-40/0004**
**Chemical Abstracts, volume 94, no. 8, February 1981, Columbus, Ohio, (US) B. Lippold et al.: "Control of drug liberation from microcapsules. part 1. Legal modification for drug transport through additive-containing lipophilic membranes", see page 362, abstract no. 52812b & Pharm. Ind. 1980, 42 (7), 745-52**

The file contains technical information submitted after the application was filed and not included in this specification

(73) Proprietor: **Key Pharmaceuticals, Inc.**
**2000 Galloping Hills Road**
**Kenilworth New Jersey 07033 (US)**

(72) Inventor: **HSIAO, Charles**
**4400 Biscayne Boulevard**
**Miami, FL 33137 (US)**
Inventor: **CHOU, Chi-Tze, Ku**
**4400 Biscayne Boulevard**
**Miami, FL 33137 (US)**

(74) Representative: **Werner, Hans-Karsten, Dr. et al**
**Deichmannhaus am Hauptbahnhof**
**D-5000 Köln 1 (DE)**

(56) References cited:
**Hanleys Condensed Chemical Dictionary. Van Nostrand Reinhold Company, N.Y. USA, 11th Edn. (1987), p936**

**Description**

Field of the invention

The present invention relates to a controlled release potassium chloride tablet. More specifically, the present invention relates to a controlled release potassium chloride tablet comprised of polymer coated crystals of KCl which is orally administered to a patient requiring potassium supplementation which tablet provides for controlled release of the potassium chloride in the gastrointestinal tract and results in substantially less irritation to the gastric mucosa.

Background of the invention

It is well known that the administration of many diuretics increases the excretion of both sodium and potassium. The acute administration of such diuretics to a patient normally causes no problems. However, chronic administration of diuretics to the patient can result in the depletion of the potassium from the patient. For example, with patients having uncomplicated hypertension, the daily administration of diuretics produces a slight reduction in plasma potassium concentration. In edematous patients, the results are more variable. Some patients suffer from substantial depletion of potassium while others fail to show any evidence of depletion. There is a high incidence of severe potassium deficiency in patients treated simultaneously with diuretics and carbenoxolone which is an agent with mineralocorticoid activity.

As can be seen from the above discussion various treatments can result in potassium depletion, i.e., hypokalemia. Potassium depletion may be accompanied by a reduced tolerance to carbohydrates and a deficiency in glycogen deposition. Further, vasopressin-resistant polyuria is a prominent symptom. A deficit of potassium appears to increase the renal synthesis of prostaglandins, which in turn can decrease the permeability to water of the distal nephron and produce a diabetes insipidus-like syndrome.

When potassium is taken along with a normal diet it is slowly absorbed from the intestinal tract. Following distribution and uptake by the cells the kidneys excrete an appropriate amount to maintain a proper balance. As a consequence of the large volume of distribution and the rapid response of the kidney, the extracellular and intracellular concentrations of the ion are normally maintained within relatively narrow limits.

When potassium is administrated as a drug, the factors that can govern the rate and extent of its distribution are of major importance. It is not possible to increase the total body content of potassium significantly above normal. However, it is very easy to raise the extracellular concentration excessively. It is the concentration in the extracellular fluid that determines life-threatening toxicity. Therefore, even though the administered potassium is eventually destined either to be excreted or taken up by the cells, knowledge of the transient concentration achieved in the plasma must govern the use of potassium as a therapeutic agent.

It is well known that large doses of potassium chloride taken orally can cause GI irritation, purging, weakness and circulatory disturbances. Since potassium depletion can cause problems for the patient as indicated above a controlled release formulation of potassium chloride which would replenish potassium in a controlled manner without the undesirable side effects is badly needed. In an attempt to meet the need of providing dosage units which can be used as potassium supplements a number of different dosage forms have been developed. For example U.S. Patent 4,352,791 discloses a composition comprised of potassium and a therapeutically acceptable salicylate salt of salicylic acid. The composition is used in potassium therapy and is useful in some respects but does not provide sufficient protection with respect to preventing gastric ulcers.

EP—A—52076 discloses potassium chloride first coated with a delayed release material such as ethylcellulose and particularly ethylcellulose mixed with a polyacrylate. This is further coated with a disintegrating agent such as polyvinylpyrrolidone. The ethylcellulose used had a lower viscosity of 9—11 mPa · s (9—11 cp).

U.S. Patent 4,340,582 discloses an enteric coated erythromycin tablet and a water-soluble nontoxic salt in the core. This core may be potassium chloride.

U.S. Patent 4,259,323 discloses a potassium chloride emulsion which includes various ingredients in an attempt to mask the bad taste of the potassium chloride. However, dosing compliance utilizing an emulsion often causes problems in that the emulsion may settle and the patient may take different amounts of the emulsion and/or different amounts of the KCl in a given amount of emulsion.

U.S. patent 4,259,315 discloses a controlled release potassium dosage form used in treating potassium deficiency. The dosage form is comprised of gelatin capsules which contain a mixture comprised of controlled release forms of micro encapsulated potassium salt and a hydrophilic surfactant.

U.S. Patent No. 4,553,399 discloses an aspirin tablet formulation comprising aspirin crystals coated with a polymeric mixture of ethylcellulose and hydroxypropylcellulose. The improved tablet is indicated as having a substantially lessened irritant effect on the gastric mucosa. The ethylcellulose used had a lower viscosity of 9—11 mPa · s (9—11 cp).

Chem. Abs. Vol. 94 (8) 52182b, Feb. 1981 discloses membranes of Eudragit[R] (a polymethacrylate) and ethylcellulose containing a water soluble polyoxyethylene additive. The transport or rate of diffusion of drugs such as chloramphenicol or carbutamide through the membranes is reported.

Sugar-coated tablets containing potassium chloride in a wax matrix (non-enteric-coated) are marketed as a slowly available potassium source. Physicians Desk Reference (1979), page 794, states "fewer bowel lesions are observed with wax-matrix tablets compared to enteric-coated potassium chloride products, but that there have been reports of upper gastrointestinal bleeding associated with the wax-matrix tablets. Use of these wax-coated products should be discontinued immediately and the possibility of bowel obstruction or perforation considered if severe vomiting, abdominal pain, distention or gastrointestinal bleeding occurs". (See USP 4,259,315).

A slow release pharmaceutical composition is disclosed within U.S. Patent 4,235,870. The composition is comprised of a combination of higher aliphatic alcohols and hydrated hydroxy-alkyl cellulose in a ratio of 2:1 to 4:1 parts by weight. The composition is intended to provide slow release of the therapeutically active compound during a predetermined period of time of from 5 to 10 hours after oral administration of the composition. However, this composition tends to remain intact and does not disintegrate, thus producing high concentrations of KCl.

Others have used surfactants to improve dissolution rate of drugs when powders agglomerate and teach the rate of dissolution is proportional to the reduction in surface tension of the gastric juice (Remington's Pharmaceutical Sciences, 15th Ed. (1973) p. 297). Others have used surfactants such as Polysorbate 20 as an ingredient interior to microcapsules during preparation of microcapsules and have discussed the adverse effect of such agents on the increased release rate of solids from the microcapsules (Luzzi et al. J. Pharm. Sci. 56(9), 1174—7 (1967). (See USP 4,259,315).

The present inventors have found that the potassium chloride dosage forms presently available are not meeting all the needs of patients requiring potassium supplementation. Accordingly, the following invention was developed.

Summary of the invention

The present invention is a controlled release potassium chloride tablet. More specifically, the invention relates to a tablet which includes potassium chloride crystals which are coated with about 9.5 to 18% by weight of a polymeric mixture. The crystals preferably have a mesh size in the range of about 30 to 50 mesh and the coating is preferably comprised of from about 9.0 to about 15 parts by weight of ethylcellulose and about 0.5 to about 3.0 parts by weight hydroxypropylcellulose. The tablets also may include a compression aid; a disintegrant and a tableting lubricant.

A primary object of the present invention is to provide a controlled release potassium chloride tablet capable of being orally administered and safely replenishing potassium in a patient suffering from potassium depletion.

Another object of the present invention is to provide such a controlled release potassium chloride tablet which when administered orally minimizes adverse side effects such as GI irritation, purging, weakness and circulatory disturbances.

Still another object of the present invention is to provide such a controlled release potassium chloride tablet which acts as a safe electrolyte replenisher.

Detailed description of the invention

The essence of the present invention is a controlled release potassium chloride tablet. The active ingredient of the tablet is the salt potassium chloride and specifically the cation potassium. The potassium cation is preferably administered to the patient in such a manner as to avoid side effects and prevent or relieve potassium depletion. The KCl tablets of the present invention may be co-administered with a diuretic.

The salt potassium chloride (KCl) occurs in nature as the mineral sylvine or sylvite. Various industrial preparations of potassium chloride exist. Further, there are numerous pharmaceutical potassium chloride preparations. The salt potassium chloride is a white crystal or crystalline powder having the following physical description: d 1.98 mp 773°C; 1 gram dissolves in 2.8 ml water; 1.8 ml boiling water; 14 ml glycerol; and 250 ml alcohol; insoluble ether and acetone.

In accordance with the present invention potassium chloride crystals having a particle size distribution ranging from 30 to 50 mesh (0.59—0.29 mm), preferably 40 mesh (0.37 mm), are utilized as "seed" crystals subjected to coating or microencapsulation and subsequently compressed into the tablet. In accordance with the present invention only minor amounts of the crystals would fall outside of the range disclosed above.

When the potassium chloride crystals having the size disclosed above are obtained they are then coated with a polymeric coating which includes ethylcellulose as a major component and hydroxypropylcellulose as a minor component. The weight ratio of the ethylcellulose to the hydroxypropylcellulose is at least 3.0. In accordance with the present invention it is contemplated that the weight ratio (ethylcellulose:hydroxypropylcellulose) can range from 3.0:1 to 30:1 with a preferred range being from 5.0:1 to 18:1, and still more preferably 9:1. Hydroxypropylcellulose (molecular weight 60,000—1,000,000 preferably 100,000) is sold under the trademark Klucel® by Hercules. The hydroxypropyl cellulose may be replaced in whole or in part with polyethylene glycol, such as that sold under the trademark Carbowax® by Union Carbide. Molecular weights of 200—8000 are useful, with 1000—6000 being preferred.

By providing the proper balance of the ethylcellulose to the hydroxypropylcellulose a polymer film can be formed on the seeds which will remain intact in the stomach (and afterwards) but which is permeable to gastric fluids, which dissolve and leach out the potassium chloride contained in the coated crystals (micro pellets). Further, these micro pellets will separate quickly upon reaching the stomach and thus avoid the accumulation of any large amount of KCl which could cause irritation.

The polymeric coating (combination of ethylcellulose and hydroxypropylcellulose) on the crystals makes up 9.5 to 18% of the total weight of the micro pellets and preferably 13.3% of the weight of the micro pellets. Lesser amounts, that is, amounts below 9% of the total weight of the micro pellets can cause the formation of bare spots on the potassium chloride crystals during the compression step, leading to undesirably rapid release of the potassium chloride in the body after oral administration. Increasing the amount of polymeric coating substantially above 18% can cause the potassium chloride to be released too slowly to be completely absorbed by the patient.

The present inventors have found it preferable to use a coating comprising ethylcellulose in an amount of 9.0 to 15% by weight based on the total weight of the micro pellets, more preferably 11.9% by weight, and hydroxypropylcellulose in an amount of 0.5 to 3.0%.

It is particularly preferred to use a higher molecular weight ethyl cellulose such as that designated as 100 and sold under the trademark Ethocel® Standard Premium 100 or Ethocel® Medium 100 by Dow Chemical. The use of higher molecular weight material like the 100 designation material limits breakage during compression. The numerical designations for ethylcellulose generally correspond to the viscosity of the product, with a higher numerical designation indicating a greater viscosity and higher molecular weight. The 100 designation corresponds to a viscosity of 85—110 mPa · s (85—110 cp) as measured in a 5% solution in an 80% toluene—20% ethanol solvent. The useful ethylcellulose designations are 7 and higher, corresponding to a viscosity of at least 6 mPa · s (6 cp), preferably more than 40 mPa · s (40 cp) (designation 45 or higher) for crystals to be compressed into tablets. The ethoxyl content can be 45—49.5%, preferably 45—46.5%. The present inventors determined that ethylcellulose 100 was preferred as compared with other ethylcellulose products as there is less breakage during compression. The lower viscosity ethylcelluloses, such as the type 10, are especially useful in making coated crystals for administration in capsules, when breakage from compression is not a problem.

Potassium chloride is normally provided in relatively large oral dosages in the range of 2 to 4 grams daily. Because of the large amount of the salt which is provided to the patient gastrointestinal irritation is common. This irritation can range from a slight discomfort to gastric ulcers. By including the crystals in the micro pellets in the manner indicated above and then compressing them in a conventional manner into tablets, the gastrointestinal irritation is alleviated or eliminated.

The individual crystals of potassium chloride having the mesh sizes indicated above are coated with the appropriate sustained release agents and compressed into tablets in a conventional manner. The tablets are coated and compressed in a manner so as to allow the tablets to disintegrate relatively quickly upon contact with an aqueous environment into the individual coated crystals, i.e., disintegration takes place in less than five minutes after oral administration.

The manufacturing process utilized applies a controlled and uniform coating permitting a more uniform dissolution as compared to a wax matrix and/or a coacervation formulation. Accordingly, the rapid disintegration and controlled dissolution of the tablets produced according to the present invention permit the peristaltic motion of the gut to distribute the coated crystals over a wide surface area. Accordingly, concentrated quantities of potassium chloride do not come in contact with the GI mucosa, thus reducing the chances for gastric ulcers. This is one of the most important features of the present invention.

The importance of potassium supplement therapy has been well established. Physicians need products for the prevention of hypokalemia during chronic diuretic therapy. Compliance is essential for patients undergoing this type of therapy. The recommended dose in most patients is 40 mmol (mEq) per day in divided doses. In accordance with currently approved labeling and 20 mmol (mEq), or 2 doses having a size of 10 mmol (mEq) should be taken twice daily in order to obtain a daily dose of 40 mmol (mEq). With the formulation provided by the present invention the tablet will include 20 mmol (mEq) so that the recommended effective amount of potassium per single dose would not be altered. The daily dose would be achieved with one tablet twice daily thus facilitating compliance due to less individual units per dose. A 20 mmol (mEq) tablet also presents to the physician a more palatable dosage form then the 20 mmol (mEq) liquid therapy and an alternative to prescribing two tablets of the same sustained release formulation.

In severe cases of hypokalemia, higher doses (60—80 mmol (mEq)) of potassium are required to reduce the loss of potassium during high dose diuretic therapy. In such cases, the physician would have available a safe higher strength tablet where, in his judgement, he is treating a patient with a compliance problem. Evidence exists that the tablet produced according to the present invention is non-irritating and non-toxic to the gastrointestinal tract.

The tablets produced in accordance with the present invention disintegrate into numerous sub-units when placed in water or placed on an aqueous food. After being disintegrated into the sub-units or micro pellets the potassium chloride of the present invention can be more easily administered to children and geriatric patients who often have difficulty in swallowing large tablets. The tablets can include conventional compression aids, e.g. microcrystalline cellulose, disintegrants, e.g. crospovidone, and lubricating agents, e.g. magnesium stearate.

Examples are prepared as indicated below:

Example 1

The potassium chloride crystals (30—50 mesh) were coated in a 15.2 cm (6'') Wurster fluidized bed column with 15% (w/w) of Ethocel® 10 and PEG 4500 E. (14:1 ratio). The Ethocel® type 10 and PEG 4500 E were dissolved in chloroform and methanol co-solvent system (4:1 ratio). The crystals were coated at 60°C inlet temperature. The spraying pressure was $1.5 \times 10^5$ Pa (1.5 bars) and the spray speed was approximately 15 ml per minute. Afterwards, 93% of the coated crystals, 6% of Avicel® PH101 (microcrystalline cellulose) and 1% of crospovidone (cross-linked polyvinylpyrrolidone) were mixed well and compressed into tablets with a Stokes DS 3 press, equipped with capsule-shape punches (0.864 cm×2.22 cm×0.218 cm) (0.34''×0.873''×0.086''). The dosage of the tablets was 20 mmol (mEq) or 1500 mg KCl.

A second batch of potassium chloride crystals was coated with 15% (w/w) of Ethocel® type 100 and PEG 4500 E (14:1 ratio) and compressed into tablets which included the excipients indicated above. All the experimental procedures were the same as mentioned above, except the type of ethylcellulose used.

A dissolution study of the coated crystals (micro pellets) was performed in deionized water.

The following table is a summary of the dissolution test:

TABLE I
Cumulative % KCl released

|  | 1 hr | 2 hr | 4 hr | 6 hr | Ethyl cellulose type |
|---|---|---|---|---|---|
| Coated crystals | 17 | 35 | 63 | 85 | Ethocel® 10 |
| Tablets | 46 | 63 | 83 | 96 | |
| Coated crystals | 12 | 59 | 86 | 96 | Ethocel® 100 |
| Tablets | 15 | 40 | 76 | 91 | |

Example 2

The potassium chloride crystals (30—50 mesh size) were coated in a 15.2 cm (6'') Wurster fluidized bed column with 15% (w/w) of Ethocel® 10, Klucel® L.F. and Mg stearate (8.5:1:0.5 ratio). The Ethocel® 10 and Klucel® L.F. were dissolved in a chloroform and methanol co-solvent system. Magnesium stearate was then added to the polymer solution to form a suspension. The suspension was stirred by a lab stirrer throughout the coating process to avoid the sedimentation of magnesium stearate. The crystals were coated at 60°C inlet temperature. The spraying pressure was $1.5 \times 10^5$ Pa (1.5 bar) and the spray speed is approximately 15 ml per minute. Afterwards, 93% of the coated crystals, 6% of Avicel® PH101 (microcrystalline cellulose) and 1% of crospovidone (cross-linked polyvinylpyrrolidone) are mixed well and compressed into capsule-shaped tablets. The dosage of the tablets was 20 mmol (20 mEq) or 1500 mg of KCl.

A second batch of potassium chloride crystals was coated with 15% (w/w) of Ethocel® 100, Klucel® L.F. and magnesium stearate (8.5:1:0.5 ratio) and compressed into tablets with the same excipients indicated above. All the experimental procedures were the same as mentioned above except the type of ethylcellulose used.

A dissolution study of the coated crystals and tablets was performed in deionized water.

The following table is a summary of the dissolution test:

TABLE II
Cumulative % KCl released

|  | 1 hr | 2 hr | 4 hr | 6 hr | Ethocel type |
|---|---|---|---|---|---|
| Coated crystals | 33 | 52 | 78 | 94 | Ethocel® 10 |
| Tablets | 52 | 69 | 86 | 97 | |
| Coated crystals | 27 | 59 | 84 | 95 | Ethocel® 100 |
| Tablets | 26 | 55 | 83 | 94 | |

In accordance with a preferred embodiment of the present invention potassium chloride tablets containing 1500 mg of potassium chloride are prepared. The potassium crystals form 68% to 86.5% by weight of these tablets and are coated with ethylcellulose (preferably Ethocel® 100) in an amount in the range of 9 to 15% by weight based on the weight of the micro pellets formed with the potassium chloride

5

crystals; 0.5 to 3% by weight of hydroxypropylcellulose based on the weight of the micro pellets; 0.5 to 2% by weight of magnesium stearate based upon the weight of the tablet; 3 to 10% by weight of microcrystalline cellulose based upon the weight of the tablet; 0.5 to 2% by weight crospovidone based upon the weight of the tablet.

Within each of these ranges it is particularly preferred for the 1500 mg tablets to include 79 weight percent of potassium chloride, 11.9% by weight of ethylcellulose (preferably Ethocel® 100), 1.4% by weight hydroxypropylcellulose, 0.7% by weight of magnesium stearate, 6% by weight of microcrystalline cellulose and 1% by weight of crospovidone.

In accordance with the present invention a clinical batch of 1500 mg tablets of potassium chloride were prepared. The tablets were comprised as shown in the following Table III.

TABLE III
Potassium chloride S.R. tablets
20 mmol (mEq)
Quantitative list of components

| Quantity/ tablets | Ingredients | Quantity/ batch | % wt. |
|---|---|---|---|
| (20 mmol (mEq)) 1500 mg | Potassium chloride, USP | 255.00 kg | 79.0 |
| 225 mg | Ethylcellulose, NF (Ethocel®, Type 100) | 38.25 kg | 11.9 |
| 27 mg | Hydroxypropylcellulose, NF (Klucel®, L.F.) | 4.50 kg | 1.4 |
| 13 mg | Magnesium stearate, NF | 2.25 kg | 0.7 |
| 114 mg | Microcrystalline Cellulose, NF (Avicel® pH 101) | 19.36 kg | 6.0 |
| 19 mg | Crospovidone, NF (Polyplasdone XL) | 3.23 kg | 1.0 |
| * | Methyl alcohol, NF (Methanol) | 168.65 kg | * |
| * | Methylene chloride, NF | 846.45 kg | * |
| 1898 mg | Totals | 322.59 kg/ 170,000 Tablets | |

*Removed during processing.

Comparative experiment

In order to demonstrate the safety of the present invention, a clinical study was carried out which compared potassium chloride tablets (20 mmol) (20 mEq) produced in accordance with the present invention with four commercial products as follows: Slow-K® (a sugar-coated wax matrix tablet from Ciba); Micro-K Extencaps®, (capsules of crystalline KCl particles coated with polymer from A. H. Robins); Kaon® Elixir (liquid potassium gluconate), and placebo.

In this particular investigator blinded study comparing the 20 mmol (mEq) KCl tablets to 4 standard preparations in a dose of 80 mmol (mEq) per day, no serious endoscopic lesions were found with the tablet of the present invention. Overall, the safety of the tablet was equal to or better than any of the comparative agents.

The present invention has been disclosed and described herein in what is considered to be its most preferred embodiments.

**Claims**

1. A pharmaceutical sustained release dosage unit in tablet form for oral administration of potassium chloride, which contains a plurality of coated potassium chloride crystals in an amount of 68% to 86.5% by weight and a coating material for each individual potassium chloride crystal which contains ethylcellulose, characterized in that

a) the amount of ethylcellulose is in the range of 9 to 15% by weight based on the total weight of the coated crystals and has a viscosity greater than 40 mPa · s (40 cp) and

b) the said coating material contains at least one member selected from hydroxypropylcellulose and polyethylene glycol in an amount in the range of 0.5% to 3% by weight based on the total weight of the coated crystals.

2. A pharmaceutical dosage unit as claimed in claim 1, wherein the tablet further contains magnesium stearate in an amount in the range of 0.5% to 2.0% by weight based on the total weight of the tablet.

3. A pharmaceutical dosage unit as claimed in claim 1 to 2, wherein the tablet further contains a microcrystalline cellulose in an amount in the range of 3 to 10% by weight based on the total weight of the tablet.

4. A pharmaceutical dosage unit as claimed in claim 1 to 3, further containing crospovidone in an amount in the range of 0.5 to 2.0% by weight based on the total weight of the tablet.

5. A pharmaceutical dosage unit as claimed in claim 1 to 4, wherein the ethylcellulose has a viscosity of from 85 to 110 mPa · s (85 to 110 cp).

6. A pharmaceutical dosage unit as claimed in claim 1 to 5, wherein the member selected from hydroxypropylcellulose and polyethylene glycol is hydroxypropylcellulose.

7. A pharmaceutical unit as claimed in claim 1 to 5, wherein the member selected from hydroxypropylcellulose and polyethylene glycol is polyethylene glycol.

**Patentansprüche**

1. Pharmazeutische Dosierungseinheit zur verzögerten Wirkstoff-Abgabe in Tablettenform zur oralen Verabreichung von Kaliumchlorid, enthaltend eine Mehrzahl von beschichteten Kaliumchlorid-Kristallen in einer Menge von 68 bis 86,5 Gew.-% und ein Ethylcellulose enthaltendes Überzugsmaterial für jeden einzelnen Kaliumchlorid-Kristall, dadurch gekennzeichnet, daß

a) die Menge der Ethylcellulose im Bereich von 9 bis 15 Gew.-%, bezogen auf das Gesamtgewicht der überzogenen Kristalle, liegt und eine Viskosität von mehr als 40 mPa · s (40 cP) hat und

b) das genannte Überzugsmaterial wenigstens eine Substanz, die aus Hydroxypropylcellulose und Polyethylenglycol ausgewählt ist, in einer Menge im Bereich von 0,5 bis 3 Gew.-%, bezogen auf das Gesamtgewicht der überzogenen Kristalle, enthält.

2. Pharmazeutische Dosierungseinheit nach Anspruch 1, worin die Tablette weiterhin Magnesiumstearat in einer Menge im Bereich von 0,5 bis 2,0 Gew.-%, bezogen auf das Gesamtgewicht der Tablette, enthält.

3. Pharmazeutische Dosierungseinheit nach Anspruch 1 oder 2, worin die Tablette weiterhin eine mikrokristalline Cellulose in einer Menge im Bereich von 3 bis 10 Gew.-%, bezogen auf das Gesamtgewicht der Tablette, enthält.

4. Pharmazeutische Dosierungseinheit nach Anspruch 1 bis 3, weiterhin enthaltend Crospovidone in einer Menge im Bereich von 0,5 bis 2,0 Gew.-%, bezogen auf das Gesamtgewicht der Tablette.

5. Pharmazeutische Dosierungseinheit nach Anspruch 1 bis 4, worin die Ethylcellulose eine Viskosität von 85 bis 110 mPa · s (85 bis 110 cP) hat.

6. Pharmazeutische Dosierungseinheit nach Anspruch 1 bis 5, worin die aus Hydroxypropylcellulose und Polyethylenglycol ausgewählte Substanz Hydroxypropylcellulose ist.

7. Pharmazeutische Dosierungseinheit nach Anspruch 1 bis 5, worin die aus Hydroxypropylcellulose und Polyethylenglycol ausgewählte Substanz Polyethylenglycol ist.

**Revendications**

1. Unité de dosage pharmaceutique à effet entretenu sous la forme de comprimé pour une administration orale de chlorure de potassium, qui contient une pluralité de cristaux de chlorure de potassium enrobés, dans une quantité de 68% à 86,5% en poids, et un matériau d'enrobage pour chaque cristal de chlorure de potassium individuel, qui contient de l'éthylcellulose, caractérisé en ce que:

a) la quantité d'éthylcellulose est dans l'intervalle de 9 à 15% en poids sur la base du poids total des cristaux enrobés et a une viscosité plus grande que 40 mPa · s (40 cp), et

b) le matériau d'enrobage précité contient au moins un membre sélectionné parmi l'hydroxypropylcellulose et le polyéthylène glycol dans une quantité dans l'intervalle de 0,5% à 3% en poids sur la base du poids total des cristaux enrobés.

2. Unité de dosage pharmaceutique selon la revendication 1, où le comprimé contient de plus du stérate de magnésium dans une quantité dans l'intervalle de 0,5% à 2,0% en poids sur la base du poids total du comprimé.

3. Unité de dosage pharmaceutique selon la revendication 1 à 2, où le comprimé contient de plus une cellulose microcristalline dans une quantité dans l'intervalle de 3 à 10% en poids sur la base du poids total du comprimé.

4. Unité de dosage pharmaceutique selon la revendication 1 à 3, contenant de plus du crospovidone dans une quantité dans l'intervalle de 0,5 à 2,0% en poids sur la base du poids total du comprimé.

5. Unité de dosage pharmaceutique selon la revendication 1 à 4, où l'éthylcellulose a une viscosité de 85 à 110 mPa · s (85 à 110 cp).

6. Unité de dosage pharmaceutique selon la revendication 1 à 5, où le membre sélectionné parmi l'hydroxypropylcellulose et le polyéthylène glycol est l'hydroxypropylcellulose.

7. Unité pharmaceutique selon la revendication 1 à 5, où le membre sélectionné parmi l'hydroxypropylcellulose et le polyéthylène glycol est le polyéthylène glycol.